# EUROPEAN PATENT SPECIFICATION

(11) **EP 4 193 964 B1**
(45) Date of publication and mention of the grant of the patent: **01.10.2025**
(21) Application number: 21853961.7
(22) Date of filing: 06.08.2021
(51) Int. Cl.: A61F 2/24

(54) **HEART VALVE ASSEMBLY AND PREPARATION METHOD THEREFOR**
HERZKLAPPENANORDNUNG UND HERSTELLUNGSVERFAHREN DAFÜR
ENSEMBLE VALVULE CARDIAQUE ET PROCÉDÉ DE PRÉPARATION ASSOCIÉ

(30) Priority: 07.08.2020 CN 202010790457
(43) Date of publication of application: 14.06.2023
(73) Proprietor: Shanqian (Zhuhai) Biomaterials Technology Co., Ltd., Zhuhai, Guangdong 519000 (CN)
(72) Inventor: HENIFORD, Ryan, Zhuhai, Guangdong 519000 (CN); XIAO, Jiahua, Zhuhai, Guangdong 519000 (CN)
(74) Representative: Schlich
(86) International application number: PCT/CN2021/111111
(87) International publication number: WO 2022/028564

(56) References cited:
- EP-A1- 3 563 804
- WO-A1-2010/020660
- CN-A- 103 006 352
- CN-A- 105 581 858
- CN-A- 107 427 355
- CN-A- 108 992 207
- CN-A- 110 090 094
- CN-A- 111 195 166
- CN-U- 212 940 074
- US-B2- 10 028 826

## Description

### TECHNICAL FIELD

The present disclosure relates to the field of medical instruments, and particularly relates to a heart valve assembly and a preparation method therefor.

### BACKGROUND

At present, there are three types of heart valves: 1, a mechanical valve; 2, a biovalve; and 3, a synthetic polymer valve.

The mechanical valves are all composed of one or more valves, these valves are mounted on an eccentric shaft, and then fixed on a safety valve seat on a heart muscle. These mechanical valves are very high in operation reliability, but are liable to cause blood flow disturbance and increase the risk of thrombosis. Therefore, a patient implanted with the mechanical valve needs to take anticoagulant drugs for life. Moreover, a valve assembly of the mechanical valve cannot be sufficiently compressed into a catheter, resulting in that the mechanical valve cannot be fed into a mounting position in a minimally invasive implanting manner of the catheter, and thus, the mechanical valve requires extremely invasive intracardiac implantation, resulting in that many elderly patients cannot use the mechanical valve due to complications.

The biovalve is a valve prosthesis produced by human organ tissues (homotransplants) or animal-derived tissues (autotransplants). The biological tissues of these valve prostheses generally can be well combined with the heart and have the additional advantage of supporting transcatheter transportation, and thus, the biovalve can overcome the above-mentioned defects of the mechanical valve and can be more widely applied. However, the biovalve is composed of organic tissues, and thus may easily naturally age and degenerate. In order to avoid natural aging and degeneration, these biological tissues commonly need to be subjected to bulk chemical treatment so as to ensure biocompatibility and prevent superficial calcification. Moreover, these biological tissues need to be mounted on one seat so as to be effectively fixed in the heart, and the arrangement of such a seat may also generate a disadvantageous flow condition inside the biovalve.

For the synthetic polymer valve, an integral valve prosthesis is produced by adopting a synthetic material, and is generally formed by adopting polyurethane or silica gel. These formed valves can effectively solve the problems related to material fatigue, and meanwhile keep natural blood flow. However, with passage of time, these synthetic polymer valves have the risk of rupture at a bent region due to the cyclic stress. In recent years, the development of the three-dimensional printing technology further increases the attempts in this field, i.e., various printable polymers are utilized to replicate the shape of a native heart valve in a more and more accurate manner. However, up to now, due to limitations of the product design and structural materials, these valves have little success clinically or commercially. Based on the cyclic stress, people have tried repeatedly to produce "full textile" heart prostheses by a weaving technology, but the full textile heart prostheses disclosed in the prior art still suffer from the problems that the prostheses have a failure due to overfatigue at the bent region, or the material and the shape cannot meet demands.

Another challenge of the current heart valve technology is the difficulty in connecting a valve leaflet material to a stent, and generally, the valve leaflet material is connected to the stent in a manual suturing manner. Such method requires highly trained and skilled technical personnel to connect various components (a valve leaflet, a suturing ring, an auxiliary valve sealing ring, and the like) of the heart valve to the stent or a frame. This process has the inherent uncertainty due to different technical personnel for suturing and the inherent subjectivity of the assembling process. EP3563804 discloses a heart valve assembly, comprising a skirt body portion which is of a tubular structure and three leaflet bodies disposed on the inner wall of the skirt body portion.

### SUMMARY

Therefore, in order to solve the problems that a prosthetic heart valve in the prior art is liable to age and it is difficult to connect a valve leaflet material to a stent, the present disclosure provides a heart valve assembly, a prosthetic valve device, and a preparation method for the heart valve assembly as defined by the claims.

According to an aspect of the present disclosure, provided is a heart valve assembly, including: a skirt body portion which is of a tubular structure, at least two leaflet bodies disposed on the inner wall of the skirt body portion, and a plurality of integrated anchoring rings arranged on the exterior of the skirt body portion. One end of the integrated anchoring ring is fixedly arranged on the exterior of the skirt body portion, and the other end of the integrated anchoring ring is a free end. The skirt body portion, the leaflet bodies, and the integrated anchoring rings form an integral valve structure.

Further, one end of the integrated anchoring ring is arranged at an edge of the skirt body portion, or arranged at a random position on the exterior of the skirt body portion in the width direction thereof, or arranged in an interweaving region between the skirt body portion and one of the leaflet bodies.

Further, the skirt body portion, the integrated anchoring ring, and the leaflet body are made of the same or different materials, which is a biocompatible polymer. The biocompatible polymer is one or more selected from ultrahigh molecular weight polyethylene (UHMWPE), polyethylene terephthalate (PET), polyether ether ketone (PEEK), thermoplastic polyurethane elastomer rubber (TPU), polyglycolic acid (PGA), polylactic acid-glycolic acid copolymer (PLGA), polylactic acid (PLA), poly-L-lactide (PLLA's), polydioxanone (PDO), polyhydroxyalkanoate (PHA's), and poly-glycerol-sebacate polyurethane (PGSU). Preferably, the skirt body portion, the integrated anchoring ring, and the leaflet body are all made of ultrahigh molecular weight polyethylene.

Further, the integrated anchoring ring has a length of 2 mm to 50 mm.

A distance between the upper and lower sides of the skirt body portion is 1 mm to 50 mm.

The heart valve assembly further includes an auxiliary valve sealing ring. The skirt body portion, the leaflet bodies, the integrated anchoring rings, and the auxiliary valve sealing ring form an integral valve structure.

Further, the skirt body portion, the integrated anchoring rings, the leaflet bodies and the auxiliary valve sealing ring are made of same or different materials.

Further, the skirt body portion is of a cylinder or cylinder-like shape.

According to an aspect of the present disclosure, provided is a prosthetic valve device, including the above heart valve assembly and further including a stent mounted on the heart valve assembly.

According to an aspect of the present disclosure, provided is a preparation method for a heart valve assembly. A fabric is formed by using a shuttle narrow-width electronic jacquard loom. The fabric includes three fabric layers, a first fabric layer thereof forms a skirt body portion, a second fabric layer thereof forms leaflet bodies, and a third fabric layer thereof forms rings which are integrated anchoring rings. These layers are interwoven together in a seamless manner at predetermined positions along the length direction of the fabric.

A method step for forming the second fabric layer includes: at a certain point along the width direction of the first fabric layer, integrally weaving the second fabric layer into the first fabric layer by using any existing weaving mode, so as to form seamless connection between two fabric layers.

Further, the shuttle narrow-width electronic jacquard loom is MEGEBA SSLMV (German).

Further, the method step for forming the second fabric layer includes: at a certain point along the width direction of the first fabric layer, integrally weaving the second fabric layer into the first fabric layer by using one of a plurality of existing weaving modes, so as to form seamless connection between two fabric layers. The existing weaving mode may be a double-layer plain orthogonal weaving mode.

Further, a specific method step for forming the integrated anchoring ring includes: guiding a weft yarn to extend in a transverse direction from an interweaving point with a selvage of the first fabric layer by a certain distance of 2 mm to 50 mm; and at a predetermined point, interweaving the weft yarn with one single warp yarn which is highly tightened to form the integrated anchoring ring. The transverse direction is the width direction of the first fabric layer.

Further, the weft yarn extends in the transverse direction from the interweaving point with the selvage of the first fabric layer by a certain distance of 2 mm.

The integral valve structure is formed in a weaving manner.

Further, two longitudinal (along the length direction of the first fabric layer) end portions of the first fabric layer may be connected by any suturing method. The suturing method includes suturing, ultrasonic welding, heat bonding, etc., and the assembly is formed to be of a cylinder or cylinder-like shape.

The one single warp yarn which is highly tightened is a concentrating line that plays the role of assisting in forming the integrated anchoring ring. When the heart valve is fixed to the stent, each anchoring ring is manipulated to a predetermined anchoring position on a stent frame by the concentrating line, thereby simplifying the connecting process.

Further, the stent is a surgical heart valve stent/self-expandable catheter transportation heart valve stent or a balloon dilatation catheter heart valve stent.

Further, the fineness of the yarns of the second to fourth fabric layers is 5 to 100 deniers.

The technical solution of the present application has the following advantages:
1. According to the heart valve assembly disclosed by the present application, the assembling process is simplified by significantly reducing the requirement on precise suturing for connecting the valve assembly to the stent. The uncertainty of the current suturing technology may also be reduced.
2. The heart valve assembly and the preparation method therefor disclosed by the present application promote application of artificial synthetic materials to surgical and transcatheter heart valves.
3. By using the heart valve assembly disclosed by the present application, the service life of a heart valve using animal-derived tissues can be prolonged.
4. The anchoring rings are integrated in a seamless manner on the exterior of the skirt body portion, which is helpful for simplifying the assembling process and reducing total time required for manufacturing the valve, so that the total cost of a manufacturing device is potentially reduced.
5. According to the heart valve assembly disclosed by the present application, the stent can be more rapidly placed and can be more rapidly assembled onto the skirt portion and the valve leaflet component, so that the accuracy and repeatability are higher, thereby reducing the requirements on well-trained assembling technical personnel.
6. The heart valve assembly disclosed by the present application can accommodate any number of leaves with any geometrical shape, and these geometrical shapes can be optimized into ideal flowing dynamics when various composite materials are used.
7. After being improved, the preparation method disclosed by the present application can be used for preparing biological textile materials required in the field of other medical instruments besides the heart valve.

### BRIEF DESCRIPTION OF THE DRAWINGS

In order to more clearly illustrate the technical solutions of the specific embodiments of the present application or the prior art, the drawings of the specific embodiments or description in the prior art will be briefly described below. It is obvious that the described drawings are only related to some embodiments of the present application, and those of ordinary skill in the art also can obtain other drawings according to the drawings, without any inventive work.
Fig.1 is a structural schematic diagram of a heart valve assembly in Embodiment 1;
Fig. 2 is a section view of three fabric layers with integrated anchoring rings;
Fig. 3 is a schematic diagram showing a concentrating line assisting in forming the integrated anchoring ring, wherein the main objective is to display how to form the anchoring ring, the reference sign 9 represents multiple fabric layers and specifically, may be a first fabric layer and/or a second fabric layer and/or a fourth fabric layer, without distinction being made to the specific fabric layers;
Fig. 4 shows a three-layer fabric including the integrated anchoring ring;
Fig. 5 is a cross section of the three-layer fabric including the integrated anchoring ring in Fig. 4, wherein the reference sign 8 represents an interweaving region among three fabric layers;
Fig. 6 shows the three-layer fabric (with the integrated anchoring ring) in which the first fabric layer is matched with an outline of a stent, wherein the reference sign 11 represents a clipping region of the first fabric layer;
Fig. 7 shows a fabric (which is integrated with an auxiliary valve sealing ring on the basis of Fig. 6) including four fabric layers in Embodiment 2, wherein the first fabric layer is matched with an outline of the stent, and the reference sign 12 represents the auxiliary valve sealing ring;
Fig. 8 is a cross section of the fabric in Fig. 7, wherein the reference sign 8 represents an interweaving region among four fabric layers;
Fig. 9 is a typical surgical heart valve assembly in the prior art;
Fig. 10 is a schematic diagram of a prosthetic valve device in Embodiment 3, and illustrates that a heart valve assembly is assembled with a stent as a whole, wherein some components are not marked;
Fig. 11 is an enlarged view of an A portion in Fig. 10; and
Fig. 12 shows the stent in Embodiment 3, wherein the circle is not a structure on the stent, but is added for more clearly displaying a predetermined anchoring point, and the reference sign 14 represents the predetermined anchoring point.

The " 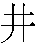 " shape in the above drawings represents textile fabrics.

### Reference signs:

1-skirt body portion; 2-leaflet body; 3-integrated anchoring ring; 4-joint; 5-concentrating line; 6-weft yarn; 7-warp yarn; 8-interweaving region between two fabric layers or among multiple fabric layers; 9-multiple fabric layers; 10-connecting region between the leaflet body and the skirt body portion; 11-clipping region; 12-auxiliary valve sealing ring; 13-stent; 14-predetermined anchoring point.

### DETAILED DESCRIPTION

The technical solutions of the present application will be described in a clearly and fully understandable way in connection with the drawings. It is obvious that the described embodiments are just a part but not all of the embodiments of the present application. Based on the embodiments of the present application, those of ordinary skill in the art can obtain other embodiments, without any inventive work, which all should be within the scope of protection of the present application.

In the description of the present application, it should be noted that directional or positional relationships indicated by terms such as "center", "upper", "lower", "left", "right", "vertical", "horizontal", "inner", and "outer" are directional or positional relationships as shown in the drawings, which are only used to facilitate description of the present application and simplify the description, but do not indicate or imply that the devices or components must have specific directions, or be constructed or operated in the specific directions, and thus cannot be construed as limiting the present application. In addition, terms such as "first", "second", and "third" are only used for description, but should not be construed as limiting or implying relative importance.

In the description of the present application, it should be noted that unless expressly specified or defined otherwise, terms "mount", "connect" and "link" should be broadly understood. For example, connect may be fixed connection, detachable connection, or integral connection, may also be mechanical connection or electrical connection, or may be direct connection, indirect connection by a medium, or internal communication between two components. Those of ordinary skill in the art can understand specific meanings of the above terms in the present application according to specific conditions.

In addition, the technical features involved in different embodiments of the present disclosure as described below may be combined with each other as long as they do not conflict with each other.

### Embodiment 1: a heart valve assembly

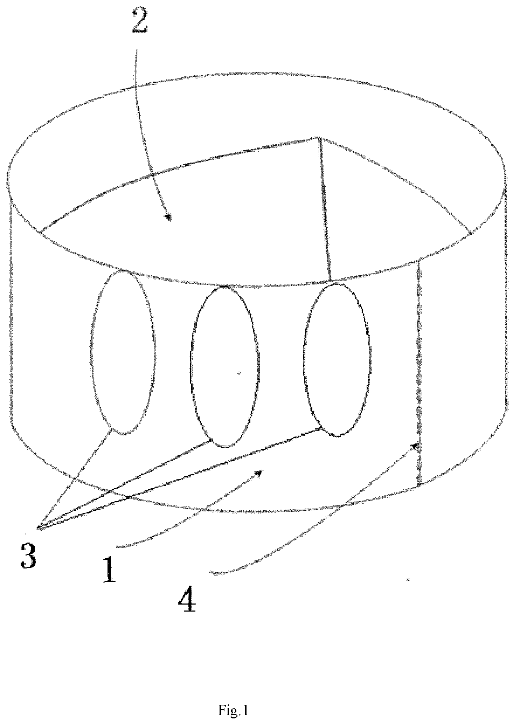

A heart valve assembly, as shown in Fig. 1, includes a skirt body portion 1 which is of a tubular structure, at least two leaflet bodies 2 connected to the inner wall of the skirt body portion 1, and a plurality of integrated anchoring rings 3 arranged on the exterior of the skirt body portion 1. The skirt body portion 1, the leaflet bodies 2, and the integrated anchoring rings 3 form an integral valve structure.

The skirt body portion 1 has selvages on upper and lower sides. Two opposite sides of the leaflet body 2 respectively are an interweaving side and a free side. The interweaving side is fixed between two selvages of the skirt body portion 1 by a weaving method, and the free side is a selvage. Two opposite sides of the integrated anchoring ring 3 respectively are an interweaving side and a free side, and the interweaving side is fixed between two selvages on the exterior of the skirt body portion 1 by the weaving method.

In the embodiment, the number of the leaflet bodies 2 is set to be 3.

The skirt body portion 1 provides a safe anchoring point for a valve leaflet, thereby preventing heart tissues around a valve from growing to the interior of the valve and disturbing functions of the leaflet and meanwhile, preventing perivalvular leakage.

The integrated anchoring ring 3 plays the role of anchoring the valve assembly onto a stent.

In the embodiment, the skirt body portion 1, the leaflet bodies 2, and the integrated anchoring rings 3 are all made of a biocompatible polymer UHMWPE.

A preparation method for the heart valve assembly (referring to Figs. 1-6) is as follows.
1) A fabric is formed by using a shuttle narrow-width electronic jacquard loom (MEGEBASSLMV). The fabric includes three fabric layers, a base layer (a first fabric layer) thereof forms the skirt body portion 1, a second fabric layer thereof forms the leaflet bodies 2, and a third fabric layer thereof comprises rings, i.e., the anchoring rings 3, which form stent anchoring components. These layers are interwoven together in a seamless manner at predetermined positions along the length direction of the fabric.

The synthetic leaflet bodies 2 are connected to the fabric base layer (the first fabric layer), and in the process of forming the first fabric layer, a weft yarn passes along two edges of the first fabric layer in the premise of not being cut off, so as to form the selvage.

The first fabric layer has a width of 1 mm to 50 mm, which depends on a length of a joint of the formed skirt body portion (a length of a connecting region between the leaflet bodies 2 and the first fabric layer). The yarn used by the first fabric layer has the fineness of 5-100 deniers, which depends on a thickness and a density required by the first fabric layer. In the embodiment, the first fabric layer has the width of 50 mm, and the yarn used by the first fabric layer has the fineness of 100 deniers.

2) At a certain point along the width direction of the first fabric layer, the second fabric layer is integrally woven into the first fabric layer by using one of a plurality of existing weaving modes (a double-layer plain orthogonal weaving mode is used in the embodiment) so as to form seamless connection between two fabric layers.

The second fabric layer forms the synthetic leaflet bodies 2 (valve leaflets), and the leaflet bodies 2 are formed by interweaving warp yarns with weft yarns.

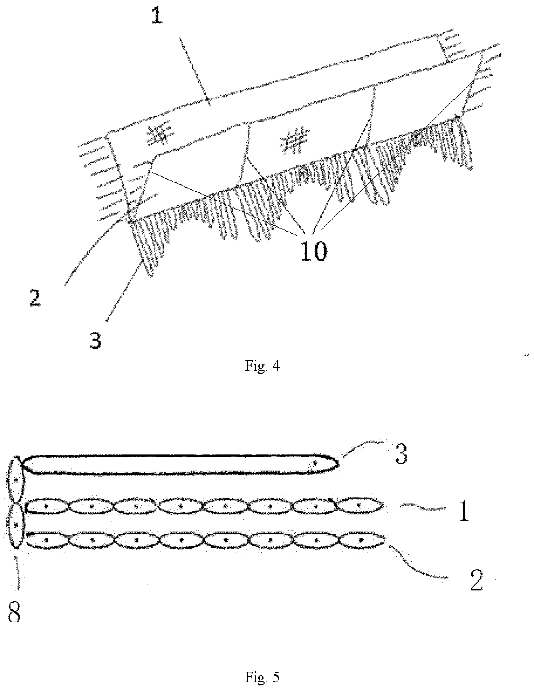

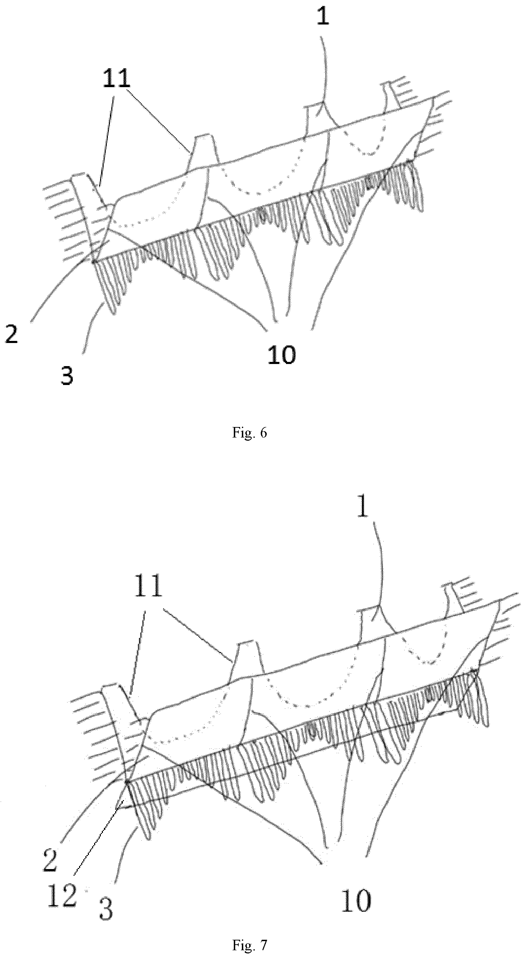

At some points along the length direction of the first fabric layer, the second fabric layer and the first fabric layer are integrally connected to form attachment points. These points can be changed in the weaving process to establish a random number of geometric figures, thereby achieving the optimum performance of the valve leaflet. In the embodiment, this figure is of a straight line shape (see Fig. 4 and Fig. 6).

In the embodiment, the shapes of the first fabric layer and the second fabric layer are as shown in Fig. 4, that is, the first fabric layer is of a rectangle shape. As an alternative embodiment, the first fabric layer can be modified by cutting to form a specific shape matched with the shape of the stent (see Fig. 6), and meanwhile, the modified skirt body portion 1 can still satisfy connection with the leaflet bodies 2.

3) On the basis of the first fabric layer and the second fabric layer, the third fabric layer is added structurally, and the third fabric layer is the integrated anchoring ring 3 formed by the weft yarns. These weft yarn rings can be interwoven with an interweaving region between the first fabric layer and the second fabric layer along one selvage of the first fabric layer, or these weft yarn rings can be interwoven with the first fabric layer along one selvage of the first fabric layer. Staggered weaving may also be carried out at different points along the width direction of the first fabric layer. Such weft yarns extend in a transverse direction (the transverse direction herein refers to the width direction of the first fabric layer) from an interweaving point with the first fabric layer and/or the second fabric layer to a predetermined length, and these rings can be anchored on anchoring points on the surface of the stent.

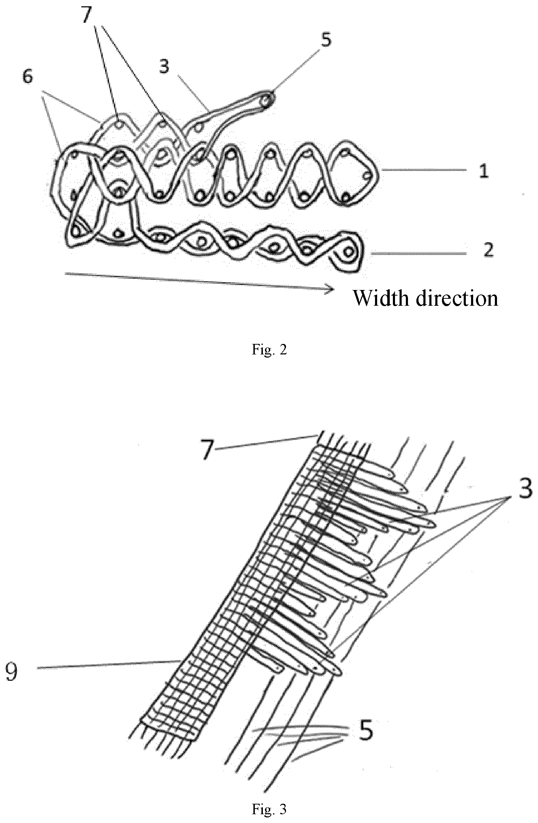

In the embodiment, a specific method step for forming the integrated anchoring ring 3 includes: guiding the weft yarn to extend in the transverse direction from the interweaving point with the selvage of the first fabric layer by a certain distance of 2 mm; and at a predetermined point, interweaving the weft yarn with one single warp yarn which is highly tightened to form one integrated anchoring ring. The one single warp yarn which is highly tightened is called as a concentrating line 5 (see Fig. 2 and Fig. 3). A plurality of integrated anchoring rings can be formed by repeating the above steps. The concentrating line may be made of fibers, yarns or steel wires, and in this embodiment, the concentrating line is made of the fibers.

As an alternative embodiment, the weft yarn extends in the transverse direction from the interweaving point with the selvage of the first fabric layer by a distance of 2 mm to 50 mm.

One single concentrating line 5 intersects with the weft yarn at a fixed point along the length direction of the first fabric layer. These concentrating lines 5 are used for assisting in forming the integrated anchoring rings, and the length of each integrated anchoring ring is finally decided by the position of the corresponding anchoring point on the stent, i.e., a final position on the stent where a prosthetic valve is attached.

Interfaces among the first fabric layer, the second fabric layer and the third fabric layer (the anchoring rings) form the seamless and integrated valve assembly. The valve assembly subsequently can be anchored onto a stent frame in a rapid and repeatable manner.

4) After the required numbers of valves, valve leaflets and anchoring rings are woven, a woven structure is taken down from the loom, and meanwhile, the concentrating lines 5 maintain sufficient tension so as to ensure that the integrated anchoring rings 3 are supported and maintained. Two longitudinal (along the length direction of the first fabric layer) end portions of the first fabric layer may be connected by any suturing method, which is shown as the joint 4 in Fig. 1. The suturing method includes suturing, ultrasonic welding, heat bonding, etc. The assembly is formed to be of a cylinder or cylinder-like shape, as shown in Fig. 1.

### Embodiment 2

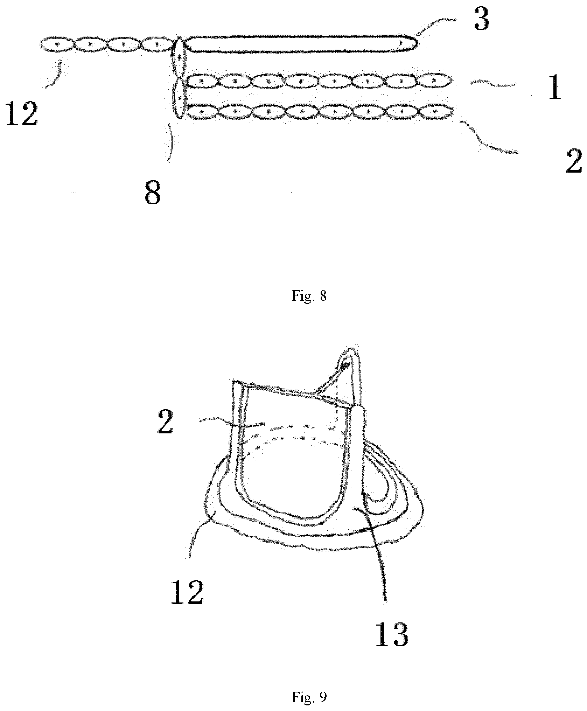

Embodiment 2 differs from Embodiment 1 in that, the heart valve assembly further includes an auxiliary valve sealing ring 12, and the auxiliary valve sealing ring 12, the skirt body portion 1, the leaflet bodies 2, and the integrated anchoring rings 3 form an integral valve structure (see Fig. 7 and Fig. 8). A fabric is formed by using the shuttle narrow-width electronic jacquard loom. The fabric includes four fabric layers; a base layer (a first fabric layer) thereof forms the skirt body portion 1; a second fabric layer thereof forms the leaflet bodies 2; a third fabric layer thereof includes rings, i.e., the anchoring rings 3, which form stent anchoring components; and a fourth layer thereof forms a suturing ring or the auxiliary valve sealing ring 12 and has an interweaving side and a free side, as shown in Fig. 7. In the embodiment, along an interweaving region between the first fabric layer and the second fabric layer, the interweaving side of the fourth fabric layer is integrally woven into the interweaving region by using one of a plurality of existing weaving modes (, a double-layer plain orthogonal weaving mode is used in the embodiment) so as to form seamless connection among the four fabric layers. The fourth fabric layer is of a square shape. These layers are interwoven together in a seamless manner at predetermined positions along the length direction of the fabric. The auxiliary valve sealing ring 12 plays the role of making extruded contact with biological tissues around a valve prosthesis so as to ensure that perivalvular leakage cannot occur during the implant positioning and the entire service life. Tissues can also be promoted to grow to the interior of the auxiliary valve sealing ring 12, which will further reduce the possibility of leakage and improve the anchoring stability of the valve with passage of time.

In the embodiment, the first fabric layer is modified by cutting to form a specific shape matched with the shape of a stent (see Fig. 7), and meanwhile, the modified skirt body portion 1 can still satisfy connection with the leaflet bodies 2.

### Embodiment 3

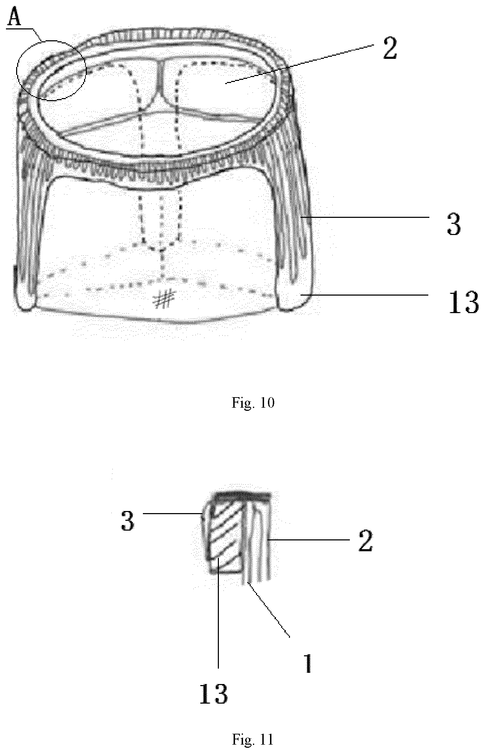

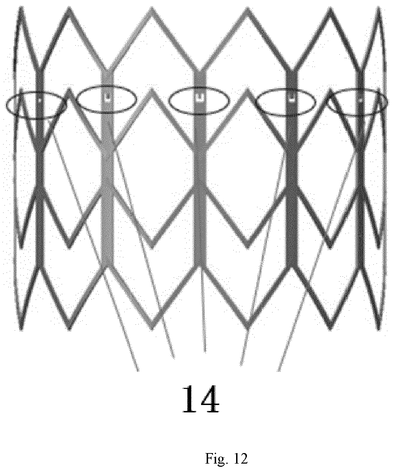

A prosthetic valve device includes the heart valve assembly manufactured in Embodiment 1 or Embodiment 2 and further includes a stent 13 mounted on the heart valve assembly. The stent is a surgical heart valve stent (which is configured for thoracotomy), a self-expandable catheter transportation heart valve stent (which is configured for a minimally invasive operation/TAVR), or a balloon dilatation catheter heart valve stent (which is configured for the minimally invasive operation/TAVR). In the embodiment, after being manufactured, the heart valve assembly can be treated by any post-treatment technology such as thermal forming, embedded molding, ultrasonic welding, solvent treatment, and scouring (washing with a solvent) to construct a final geometrical shape; and after construction, the heart valve assembly and the stent can be fixed. A method step for fixing the stent on the heart valve assembly depends on the geometrical shape of the stent and the structure of the heart valve assembly, and fixing between the heart valve assembly and the stent is implemented by the anchoring rings (see Fig. 10 to Fig. 12). In the embodiment, the method step for fixing the stent 13 on the heart valve assembly includes: hanging free ends of the anchoring rings at predetermined anchoring points 14 of the stent; and manipulating the respective integrated anchoring rings to predetermined anchoring positions on a stent frame by utilizing concentrating lines, and then removing the concentrating lines 5. The connecting process is simplified.

By adopting the heart valve assembly provided in the present application, the stent can be more conveniently aligned and positioned, so that mounting can be more accurate, simpler and more convenient, and the subjectivity is lower.

Once the heart valve assembly is anchored onto the stent, a method for fixing the stent onto other components will depend on the shape of the stent, expected application (i.e., an aortic valve, a mitral valve, and the like) and an operating method (i.e., transcatheter transportation, open heart surgery, and the like). The rest of valve components can be fixed by various methods including, but not limited to, suturing, welding, selection placement of an adhesive, the design of a "pocket" at the skirt body portion, so as to reinforce anchoring with the stent.

Obviously, the foregoing embodiments merely are examples for clear illustration, but not intended to limit the embodiments. Those of ordinary skill in the art also can make other different forms of variations or changes on the basis of the illustration above. It is unnecessary and impossible to enumerate all embodiments herein. The apparent variations or changes made on the basis of the embodiments of the present disclosure are still within the scope of protection of the present application.

## Claims

1. A heart valve assembly, comprising: a skirt body portion (1) which is of a tubular structure, at least two leaflet bodies (2) disposed on the inner wall of the skirt body portion, and a plurality of integrated anchoring rings (3) arranged on the exterior of the skirt body portion; one end of the integrated anchoring ring being fixedly arranged on the exterior of the skirt body portion, and the other end of the integrated anchoring ring being a free end; and the skirt body portion, the leaflet bodies and the integrated anchoring rings forming an integral valve structure.

2. The heart valve assembly of claim 1, wherein one end of the integrated anchoring ring is arranged at an edge of the skirt body portion, or arranged at a random position on the exterior of the skirt body portion in the width direction thereof, or arranged in an interweaving region between the skirt body portion and one of the leaflet bodies.

3. The heart valve assembly of claim 1 or 2, wherein the skirt body portion, the integrated anchoring ring and the leaflet body are made of the same or different materials, which is a biocompatible polymer; preferably, the biocompatible polymer is one or more selected from ultrahigh molecular weight polyethylene, polyethylene terephthalate, polyether ether ketone, thermoplastic polyurethane elastomer rubber, polyglycolic acid, polylactic acid-glycolic acid copolymer, polylactic acid, poly-L-lactide, polydioxanone, polyhydroxyalkanoate, and poly-glycerol-sebacate polyurethane; and more preferably, the skirt body portion, the integrated anchoring ring and the leaflet body are all made of ultrahigh molecular weight polyethylene.

4. The heart valve assembly of claim 1 or 2, wherein the integrated anchoring ring has a length of 2 mm to 50 mm; and preferably, a distance between the upper and lower sides of the skirt body portion is 1 mm to 50 mm.

5. The heart valve assembly of any one of claims 1-4, further comprising an auxiliary valve sealing ring, wherein the skirt body portion, the leaflet bodies, the integrated anchoring rings and the auxiliary valve sealing ring form an integral valve structure.

6. A preparation method for a heart valve assembly, wherein a fabric is formed by using a shuttle narrow-width electronic jacquard loom; the fabric comprises three fabric layers, a first fabric layer thereof forms a skirt body portion (1), a second fabric layer thereof forms leaflet bodies (2), and a third fabric layer thereof forms rings which are integrated anchoring rings (3); and these layers are interwoven together in a seamless manner at predetermined positions along the length direction of the fabric.

7. The preparation method for the heart valve assembly of claim 6, wherein a method step for forming the second fabric layer comprises: at a certain point along the width direction of the first fabric layer, integrally weaving the second fabric layer into the first fabric layer by using any existing weaving mode, so as to form seamless connection between two fabric layers.

8. The preparation method for the heart valve assembly of claim 6 or 7, wherein a specific method step for forming the integrated anchoring ring comprises: guiding a weft yarn to extend in a transverse direction from an interweaving point with a selvage of the first fabric layer by a certain distance of 2 mm to 50 mm; and at a predetermined point, interweaving the weft yarn with one single warp yarn which is highly tightened to form the integrated anchoring ring; wherein the transverse direction is the width direction of the first fabric layer.

9. The preparation method for the heart valve assembly of claim 8, wherein the weft yarn extends in the transverse direction from the interweaving point with the selvage of the first fabric layer by a certain distance of 2 mm.

10. A prosthetic valve device, comprising the heart valve assembly of any one of claims 1-5 or a heart valve assembly produced by the preparation method of any one of claims 6-9, and further comprising a stent mounted on the heart valve assembly.

## Patentansprüche

1. Herzklappenanordnung, umfassend: einen Schürzenkörperabschnitt (1), der von einer rohrförmigen Struktur ist, mindestens zwei Segelkörper (2), die an der Innenwand des Schürzenkörperabschnitts vorgesehen sind, und eine Vielzahl von integrierten Verankerungsringen (3), die an der Außenseite des Schürzenkörperabschnitts angeordnet sind; wobei ein Ende des integrierten Verankerungsrings fest an der Außenseite des Schürzenkörperabschnitts angeordnet ist und das andere Ende des integrierten Verankerungsrings ein freies Ende ist; und wobei der Schürzenkörperabschnitt, die Segelkörper und die integrierten Verankerungsringe eine einstückige Klappenstruktur bilden.

2. Herzklappenanordnung nach Anspruch 1, wobei ein Ende des integrierten Verankerungsrings an einer Kante des Schürzenkörperabschnitts angeordnet ist oder an einer zufälligen Position an der Außenseite des Schürzenkörperabschnitts in der Breitenrichtung davon angeordnet ist oder in einem Verwebungsbereich zwischen dem Schürzenkörperabschnitt und einem der Segelkörper angeordnet ist.

3. Herzklappenanordnung nach Anspruch 1 oder 2, wobei der Schürzenkörperabschnitt, der integrierte Verankerungsring und der Segelkörper aus dem gleichen oder verschiedenen Materialien gefertigt sind, bei denen es sich um ein biokompatibles Polymer handelt; wobei vorzugsweise das biokompatible Polymer eines oder mehrere ist, die aus Polyethylen mit ultrahohem Molekulargewicht, Polyethylenterephthalat, Polyetheretherketon, thermoplastischem Polyurethanelastomer-Kautschuk, Polyglykolsäure, Polymilchsäure-Glykolsäure-Copolymer, Polymilchsäure, Poly-L-lactid, Polydioxanon, Polyhydroxyalkanoat und Polyglycerin-Sebacat-Polyurethan ausgewählt sind; und wobei noch bevorzugter der Schürzenkörperabschnitt, der integrierte Verankerungsring und der Segelkörper alle aus Polyethylen mit ultrahohem Molekulargewicht gefertigt sind.

4. Herzklappenanordnung nach Anspruch 1 oder 2, wobei der integrierte Verankerungsring eine Länge von 2 mm bis 50 mm aufweist; und vorzugsweise ein Abstand zwischen der oberen und der unteren Seite des Schürzenkörperabschnitts 1 mm bis 50 mm beträgt.

5. Herzklappenanordnung nach einem der Ansprüche 1 bis 4, ferner umfassend einen Hilfsklappendichtring, wobei der Schürzenkörperabschnitt, die Segelkörper, die integrierten Verankerungsringe und der Hilfsklappendichtring eine einstückige Klappenstruktur bilden.

6. Herstellungsverfahren für eine Herzklappenanordnung, wobei ein Gewebe durch Verwendung eines elektronischen Schiffchen-Jacquardwebstuhls für schmale Breiten gebildet wird; wobei das Gewebe drei Gewebelagen umfasst, wobei eine erste Gewebelage davon einen Schürzenkörperabschnitt (1) bildet, eine zweite Gewebelage davon Segelkörper (2) bildet und eine dritte Gewebelage davon Ringe bildet, die integrierte Verankerungsringe (3) sind; und wobei diese Lagen an vorbestimmten Positionen entlang der Längsrichtung des Gewebes nahtlos miteinander verwoben werden.

7. Herstellungsverfahren für die Herzklappenanordnung nach Anspruch 6, wobei ein Verfahrensschritt zum Bilden der zweiten Gewebelage Folgendes umfasst: an einem bestimmten Punkt entlang der Breitenrichtung der ersten Gewebelage, integrales Weben der zweiten Gewebelage in die erste Gewebelage durch Verwendung eines beliebigen vorhandenen Webemodus, um eine nahtlose Verbindung zwischen zwei Gewebelagen zu bilden.

8. Herstellungsverfahren für die Herzklappenanordnung nach Anspruch 6 oder 7, wobei ein spezifischer Verfahrensschritt zum Bilden des integrierten Verankerungsrings Folgendes umfasst: Führen eines Schussfadens, um sich in einer Querrichtung von einem Verwebungspunkt mit einer Webkante der ersten Gewebelage um einen bestimmten Abstand von 2 mm bis 50 mm zu erstrecken; und an einem vorbestimmten Punkt Verweben des Schussfadens mit einem einzelnen Kettfaden, der stark angezogen ist, um den integrierten Verankerungsring zu bilden; wobei die Querrichtung die Breitenrichtung der ersten Gewebelage ist.

9. Herstellungsverfahren für die Herzklappenanordnung nach Anspruch 8, wobei sich der Schussfaden in der Querrichtung von dem Verwebungspunkt mit der Webkante der ersten Gewebelage um einen bestimmten Abstand von 2 mm erstreckt.

10. Prothesenklappenvorrichtung, umfassend die Herzklappenanordnung nach einem der Ansprüche 1-5 oder eine Herzklappenanordnung, die durch das Herstellungsverfahren nach einem der Ansprüche 6-9 produziert ist, und ferner umfassend einen Stent, der auf der Herzklappenanordnung montiert ist.

## Revendications

1. Ensemble valvule cardiaque, comprenant : une partie corps de jupe (1) qui est d'une structure tubulaire, au moins deux corps de feuillet (2) disposés sur la paroi interne de la partie corps de jupe, et une pluralité d'anneaux d'ancrage intégrés (3) agencés sur l'extérieur de la partie corps de jupe ; une extrémité de l'anneau d'ancrage intégré étant agencée de manière fixe sur l'extérieur de la partie corps de jupe, et l'autre extrémité de l'anneau d'ancrage intégré étant une extrémité libre ; et la partie corps de jupe, les corps de feuillet et les anneaux d'ancrage intégrés formant une structure de valvule intégrale.

2. Ensemble valvule cardiaque selon la revendication 1, une extrémité de l'anneau d'ancrage intégré étant agencée au niveau d'un bord de la partie corps de jupe, ou agencée au niveau d'une position aléatoire sur l'extérieur de la partie corps de jupe suivant le sens de la largeur de celle-ci, ou agencée dans une zone d'entrelacement entre la partie corps de jupe et l'un des corps de feuillet.

3. Ensemble valvule cardiaque selon la revendication 1 ou 2, ladite partie corps de jupe, ledit anneau d'ancrage intégré et ledit corps de feuillet étant constitués du même matériau ou de matériaux différents, qui est un polymère biocompatible ; de préférence, ledit polymère biocompatible étant un ou plusieurs polymères choisis parmi le polyéthylène de poids moléculaire ultra-élevé, le polyéthylène téréphtalate, la polyéther éther cétone, le caoutchouc élastomère de polyuréthane thermoplastique, l'acide polyglycolique, le copolymère acide polylactique-acide glycolique, l'acide polylactique, le poly-L-lactide, la polydioxanone, le polyhydroxyalcanoate et le poly-glycérol-sébacate polyuréthane ; et plus préférablement, ladite partie corps de jupe, ledit anneau d'ancrage intégré et ledit corps de feuillet étant tous constitués de polyéthylène de poids moléculaire ultra-élevé.

4. Ensemble valvule cardiaque selon la revendication 1 ou 2, ledit anneau d'ancrage intégré présentant une longueur de 2 mm à 50 mm ; et de préférence, la distance entre les côtés supérieur et inférieur de la partie corps de jupe étant de 1 mm à 50 mm.

5. Ensemble valvule cardiaque selon l'une quelconque des revendications 1 à 4, comprenant en outre une bague d'étanchéité de valvule auxiliaire, ladite partie corps de jupe, lesdits corps de feuillet, lesdits anneaux d'ancrage intégrés et ladite bague d'étanchéité de valvule auxiliaire formant une structure de valvule intégrale.

6. Procédé de préparation pour un ensemble valvule cardiaque, un tissu étant formé à l'aide d'un métier à tisser Jacquard électronique à largeur étroite à navette ; ledit tissu comprenant trois couches de tissu, une première couche de tissu de celui-ci formant une partie corps de jupe (1), une deuxième couche de tissu de celui-ci formant des corps de feuillet (2), et une troisième couche de tissu de celui-ci formant des anneaux qui sont des anneaux d'ancrage intégrés (3) ; et ces couches étant entrelacées ensemble d'une manière sans couture au niveau de positions prédéfinies dans le sens de la longueur du tissu.

7. Procédé de préparation pour l'ensemble valvule cardiaque selon la revendication 6, une étape de procédé pour la formation de la deuxième couche de tissu comprenant : au niveau d'un certain point dans le sens de la largeur de la première couche de tissu, le tissage intégral de la deuxième couche de tissu dans la première couche de tissu en utilisant un quelconque mode de tissage existant, de manière à former une liaison sans couture entre deux couches de tissu.

8. Procédé de préparation pour l'ensemble valvule cardiaque selon la revendication 6 ou 7, une étape de procédé spécifique pour la formation de l'anneau d'ancrage intégré comprenant : le guidage d'un fil de trame de manière à ce qu'il s'étende suivant le sens transversal à partir d'un point d'entrelacement avec une lisière de la première couche de tissu d'une certaine distance de 2 mm à 50 mm ; et au niveau d'un point prédéfini, l'entrelacement du fil de trame avec un fil de chaîne unique qui est fortement serré de manière à former l'anneau d'ancrage intégré ; ledit sens transversal étant le sens de la largeur de la première couche de tissu.

9. Procédé de préparation pour l'ensemble valvule cardiaque selon la revendication 8, ledit fil de trame s'étendant suivant le sens transversal à partir du point d'entrelacement avec la lisière de la première couche de tissu d'une certaine distance de 2 mm.

10. Dispositif de valvule prothétique, comprenant l'ensemble valvule cardiaque selon l'une quelconque des revendications 1 à 5 ou un ensemble valvule cardiaque produit par le procédé de préparation selon l'une quelconque des revendications 6 à 9, et comprenant en outre une endoprothèse montée sur l'ensemble valvule cardiaque.
